(19) Europäisches Patentamt / European Patent Office / Office européen des brevets

(11) **EP 1 363 601 B1**

(12) **EUROPÄISCHE PATENTSCHRIFT**

(45) Veröffentlichungstag und Bekanntmachung des Hinweises auf die Patenterteilung:
**06.06.2007 Patentblatt 2007/23**

(21) Anmeldenummer: **02701290.5**

(22) Anmeldetag: **21.02.2002**

(51) Int Cl.:
**A61K 9/127** *(2006.01)*

(86) Internationale Anmeldenummer:
**PCT/EP2002/001880**

(87) Internationale Veröffentlichungsnummer:
**WO 2002/066012 (29.08.2002 Gazette 2002/35)**

(54) **AMPHOTERE LIPOSOMEN UND VERWENDUNG DIESER**

AMPHOTERIC LIPOSOMES AND THE USE THEREOF

LIPOSOMES AMPHOTERES ET LEUR UTILISATION

(84) Benannte Vertragsstaaten:
**AT BE CH CY DE DK ES FI FR GB GR IE IT LI LU MC NL PT SE TR**

(30) Priorität: **21.02.2001 DE 10109897**

(43) Veröffentlichungstag der Anmeldung:
**26.11.2003 Patentblatt 2003/48**

(73) Patentinhaber: **novosom AG**
**06120 Halle (DE)**

(72) Erfinder:
- **PANZNER, Steffen**
  **06114 Halle (DE)**
- **FANKHÄNEL, Stefan**
  **04451 Borsdorf (DE)**
- **ESSLER, Frank**
  **55131 Mainz (DE)**
- **PANZNER, Cornelia**
  **06114 Halle (DE)**
- **Herr Dr. Gerold Endert**
  **06114 Halle (DE)**

(74) Vertreter: **Ziebig, Marlene et al**
**Anwaltskanzlei**
**Gulde Hengelhaupt Ziebig & Schneider**
**Wallstrasse 58/59**
**10179 Berlin (DE)**

(56) Entgegenhaltungen:
**WO-A-00/28972          WO-A-00/59474**
**WO-A-97/39019**

- **DATABASE CA [Online] CHEMICAL ABSTRACTS SERVICE, COLUMBUS, OHIO, US; HAFEZ, ISMAIL M. ET AL: "Tunable pH- sensitive liposomes composed of mixtures of cationic and anionic lipids" retrieved from STN Database accession no. 133:355087 CA XP002204268 & BIOPHYSICAL JOURNAL (2000), 79(3), 1438-1446 , 2000,**
- **HITOYUKI FUKUDA ET AL: "BILAYER-FORMING ION-PAIR AMPHIPHILES FROM SINGLE-CHAIN SURFACTANTS" JOURNAL OF THE AMERICAN CHEMICAL SOCIETY, AMERICAN CHEMICAL SOCIETY, WASHINGTON, DC, US, Bd. 112, Nr. 4, 14. Februar 1990 (1990-02-14), Seiten 1635-1637, XP000100893 ISSN: 0002-7863**
- **R. LEVENTIS ET AL.: "pH-dependent stability and fusion of liposomes combining protonatable double-chain amphiphiles with phosphatidylethanolamine" BIOCHEMISTRY, Bd. 26, Nr. 12, 1987, Seiten 3267-3276, XP002025621 Easton (US)**
- **ZI CHEN LI ET AL: "SYNTHESIS OF CHOLESTEROL DERIVATIVES WITH AMINO ACID AS HYDROPHILIC GROUP AND THE VESICLES PREPARED THEREFROM" CHINESE CHEMICAL LETTERS, XX, XX, Bd. 12, Nr. 10, 1999, Seiten 1007-1010, XP008005314 ISSN: 1001-8417**

EP 1 363 601 B1

**Beschreibung**

Die Erfindung betrifft amphotere Liposomen, die zugleich positive und negative membranständige oder membranbildende Ladungsträger umfassen sowie die Verwendung dieser Liposomen.

Unter dem Begriff der Lipide werden drei Klassen von Naturstoffen zusammengefasst, die sich aus biologischen Membranen isolieren lassen: Phospholipide, Sphingolipide und Cholesterol mit seinen Derivaten. Dazu gehören aber auch synthetisch erzeugte Stoffe mit ähnlicher Charakteristik. Hier seien die Diacylglycerole, Dialkylglycerole, 3-Amino-1,2-Propandiolester oder -ether oder auch die N,N-Dialkylamine stellvertretend genannt.

Von technischem Interesse sind diese Substanzen bei der Herstellung von Liposomen. Diese Liposomen lassen sich unter anderem als Container für Wirkstoffe bei pharmazeutischen Zubereitungen einsetzen. Wünschenswert ist dabei eine effiziente und stabile Verpackung des Cargos, Verträglichkeit mit Körperflüssigkeiten und eine kontrollierbare und gegebenenfalls ortsspezifische Freisetzung des Inhalts.

Beide Anforderungen sind nachteilhafterweise schwer zu vereinen: Je stabiler und dichter die Verpackung ist, desto schwerer gibt sie den eingeschlossenen Wirkstoff wieder frei. Aus diesem Grund wurden Liposomen entwickelt, die ihre Eigenschaften als Reaktion auf einen äußeren Reiz verändern.

Bekannt sind thermosensible und pH-sensitive Liposomen. Die pH-sensitiven Liposomen sind von besonderem Interesse, da dieser Parameter sich auch unter physiologischen Umständen, etwa bei der endozytotischen Aufnahme eines Liposoms in Zellen oder bei der Passage des Magen-Darm-Trakts, ändern kann. Nach dem Stand der Technik umfassen pH-sensitive Liposomen insbesondere Cholesterolhemisuccinat (CHEMS).

Cholesterolhemisuccinat wird in Mischung mit Phosphatidylethanolamin zur Herstellung pH-sensitiver Liposomen verwendet (Tachibana et al.(1998); BBRC 251: 538-544, US4891208). Solche Liposomen können von Zellen endozytiert werden und vermögen auf diesem Weg Cargomoleküle in das Innere von Zellen zu transportieren, ohne die Integrität der zellulären Membran zu verletzen.

Ein wesentlicher Nachteil des CHEMS ist dessen anionischer Charakter. Die damit hergestellten Liposomen besitzen eine negative Gesamtladung und werden nur mit geringer Effizienz von Zellen aufgenommen. Trotz des oben beschriebenen Transfermechanismus eignen sie sich daher kaum für den Eintransport von Makromolekülen in Zellen.

Für den Eintransport von Wirkstoffen in Zellen (Transfektion) werden fachgemäß kationische Liposomen verwendet, die über eine möglichst hohe und konstante Oberflächenladung verfügen. Die positive Gesamtladung solcher Partikel führt zu einer elektrostatischen Anheftung an Zellen und in der Folge zu einem effizienten Eintransport. Der Einsatz dieser Verbindungen und der damit hergestellten Liposomen bleibt aber auf Anwendungen in vitro oder ex vivo beschränkt, da solche positiv geladenen Liposomen mit Serumbestandteilen unkontrollierte Aggregate bilden.

BIOPHYSICAL JOURNAL (2000), 79(3), pp. 1438-1446, offenbart pH-sensitive Liposomen bestehend aus einer Kombination von anionischen und kationischen Lipiden, z. b. CHEMS und DODAC bzw. DOPA und DC-Chol, ohne den Zusatz von Neutrallipiden.

In der WO 97 39019 A werden Liposomen beschrieben, die z. B. aus DOPE und DC-Chol hergestellt werden. Die in der WO 97 39019 A offenbarten Liposomen besitzen einen isoelektrischen Punkt, der größer 8 ist, da DOPE bis pH 8 ungeladen vorliegt und DC-Chol bis ca. pH 9 geladen ist.

Die WO 00 59474 A offenbart Liposomen, die aus einer Kombination von konventionellen Lipiden, wie z. B. DOPE und einem neuen Lipid, das einen positiven und einen negativen Ladungsträger aufweist, hergestellt werden, wobei der negative Ladungsträger von einer Disulfidbindung, welche als chemischer "Schalter" dient, mit dem Rest des Moleküls verbunden wird. Die hier beschriebenen Liposomen können aufgrund ihres irreversiblen chemischen "Schalters" die Umladung nicht rückgängig machen.

BIOCHEMISTRY, Bd. 26, Nr. 12, 1987, pp. 3267 - 3276, beschreibt Liposomen aus einem Phospholipid, z. B. DOPE und einem schwach anionischen Lipid.

Nachteilig bei den im Stand der Technik verfügbaren pH-sensitiven Liposomen ist die Beschränkung auf sehr wenige pK-Werte, zumeist den der Carboxygruppe im Cholesterolhemisuccinat (ca. 4,5). Ein weiterer Nachteil der Verbindungen ist die Beschränkung auf negative Ladungsträger. Diese eignen sich nicht zur effizienten Bindung von Nukleinsäuren und oft auch nicht für Proteine.

Kationische Liposomen zeigen eine gute Bindung von Nukleinsäuren und Proteinen und sind in der Lage, diese Wirkstoffe in Zellen einzubringen. Nachteilhafterweise sind sie nicht für in vivo-Applikationen einsetzbar.

Es bestand daher die Aufgabe, liposomale Strukturen herzustellen, die

    i) einen effizienten Einschluß von Wirkstoffen erlauben,
    ii) diese Wirkstoffe in biologische Zellen tranportieren können,
    iii) kompatibel mit dem Einsatz unter in vivo-Bedingungen sind
    iv) einfach und preiswert herzustellen sind.

Die erfindungsgemäße Aufgabe wird durch amphotere Liposomen gemäß Ansprüche 1 und 3 gelöst. Die amphoteren Liposomen umfassen mindestens einen positiven und mindestens einen davon verschiedenen negativen Ladungsträger, wobei der isoelektrische Punkt der Liposomen zwischen 4 und 8 liegt, wobei die Liposomen mit einer pH-abhängig wechselnden Ladung hergestellt werden.

Liposomale Strukturen mit den gewünschten Eigenschaften entstehen beispielsweise, wenn bei einem niedrigen pH-Wert die Menge der membranbildenden oder membranständigen kationischen Ladungsträger die der anionischen überwiegt und sich bei einem höheren pH-Wert diese Verhältnisse jedoch umkehren. Das ist immer dann der Fall, wenn die ionisierbaren Komponenten einen pKa-Wert im Bereich zwischen 4 und 9 haben. Alle kationischen Ladungsträger werden dann bei einem sinkenden pH des Mediums stärker aufgeladen, alle anionischen Ladungsträger verlieren ihre Ladung.

Im Zusammenhang mit der Erfindung sollen folgende Abkürzungen verwendet werden:

| | |
|---|---|
| CHEMS | Cholesterolhemisuccinat |
| PC | Phosphatidylcholin |
| PE | Phosphatidylethanolamin |
| PS | Phosphatidylserin |
| PG | Phosphatidylglycerol |
| Hist-Chol | Histidinylcholesterolhemisuccinat |

Die membranbildenden oder membranständigen Ladungsträger haben die folgende allgemeine Struktur eines Amphiphils:

Ladungsgruppe - Membrananker

Als Membrananker kommen die aus der Natur bekannten Systeme oder deren technische abgewandelten Formen in Frage. Dazu gehören insbesondere die Diacylglycerole, Diacylphosphoglycerole (Phospholipide) und Sterole, aber auch die Dialkylglycerole, die Dialkyl oder Diacyl-1-Amino-2,3-Propandiole, langkettige Alkyle oder Acyle mit 8 bis 25 C-Atomen, Sphingolipide, Ceramide und andere mehr. Diese Membrananker sind fachgemäß und im Stand der Technik bekannt.

Die Ladungsgruppen, die sich mit diesen Ankern kombinieren lassen, können in folgende 6 Gruppen eingeteilt werden:

Stark kationisch, pKa>9, positive Nettoladung: Ihrer chemischen Natur nach sind das beispielsweise Ammonium-, Amidinium-, Guanidinium- oder Pyridiniumgruppen oder primäre, sekundäre oder tertiäre Aminofunktionen.

Schwach kationisch, pKa<9, positive Nettoladung: Ihrer chemischen Natur nach sind das insbesondere Stickstoffbasen wie beispielsweise Piperazine, Imidazole und Morpholine, Purine oder Pyrimidine. Bevorzugt sind solche Molekülfragmente, wie sie in biologischen Systemen vorkommen, also beispielsweise 4-Imidazole (Histamin), 2-,6- oder 9-Purine (Adenine,Guanine, Adenosine oder Guanosine), 1-, 2-oder 4-Pyrimidine (Uracile, Thymine, Cytosine, Uridine, Thymidine, Cytidine) oder auch Pyridin-3-carbonsäuren (Nicotinsäureester oder -amide).

Stickstoffbasen mit bevorzugten pKa-Werten entstehen auch durch einfache oder mehrfache Substitution des Stickstoffatoms mit Niederalkanhydroxylen, etwa Hydroxymethyl- oder Hydroxyethylgruppen. Geeignete organische Basen aus dieser Gruppe sind beispielsweise Aminopropandiole, Triethanolamine, Tris-(hydroxymethyl)methylamine, Bis-(hydroxymethyl) methylamine, Tris-(hydroxyethyl)methylamine, Bis-(hydroxyethyl)methylamine oder die entsprechend substituierten Ethylamine.

Neutral oder im pH-Bereich zwischen 4 und 9 zwitterionisch: Ihrer chemischen Natur nach sind das neutrale Gruppen wie Hydroxyle, Amide, Thiole oder Zwitterionen aus einer starken kationischen und einer starken anionischen Gruppe wie beispielsweise das Phosphocholin oder Aminocarbonsäuren, Aminosulfonsäuren, Betaine oder andere Strukturen.

Schwach anionisch, pKa>4, negative Nettoladung: Ihrer chemischen Natur nach sind das besonders die Carbonsäuren. Dazu gehören die aliphatischen, geradkettigen oder verzweigten Mono-, Di- oder Tricarbonsäuren mit bis zu 12 C-Atomen und 0, 1 oder 2 ethylenisch ungesättigten Bindungen. Carbonsäuren mit einem geeigneten Verhalten findet man auch als Substituenten aromatischen Systeme.

Andere anionische Gruppen sind dissoziierbare Hydroxyle oder Thiole, wie sie in der Ascorbinsäure, dem N-substituierten Alloxan, der N-substituierten Barbitursäure, im Veronal, dem Phenol oder als Thiolgruppe vorkommen.

Stark kationisch, pKa<4, negative Nettoladung: Ihrer chemischen Natur nach sind das funktionelle Gruppen wie beispielsweise die Sulfonsäureester oder Phosphorsäureester.

Amphotere Ladungsträger, pI zwischen 4,5 und 8,5, positive Nettoladung unterhalb des pI, negative Nettoladung oberhalb des pI: Ihrer chemischen Natur nach sind diese Ladungsträger aus zwei oder mehreren Fragmenten der oben genannten Gruppen zusammengesetzt. Es ist für die Ausführung der Erfindung zunächst nicht wesentlich, ob sich die geladenen Gruppen auf ein und demselben Membrananker befinden oder ob sich diese Gruppen auf verschiedenen Ankern befinden. Besonders bevorzugt für die Ausführung der Erfindung sind amphotere Ladungsträger mit einem pI zwischen 5 und 7.

Stark kationische Verbindungen sind beispielsweise:

DC-Chol 3-β-[N-(N',N'-dimethylethane) carbamoyl]cholesterol
TC-Chol 3-β-[N-(N',N', N'-trimethylaminoethane) carbamoyl] cholesterol
BGSC Bis-guanidinium-spermidine-cholesterol
BGTC Bis-guanidinium-tren-cholesterol,
DOTAP (1,2-dioleoyloxypropyl)-N,N,N-trimethylammonium chlorid
DOSPER (1,3-dioleoyloxy-2-(6-Carboxy-spermyl)-propylamid)
DOTMA (1,2-dioleyloxypropyl)-N,N,N-trimethylammonium chlorid) (Lipofectin®)
DORIE (1,2-dioleyloxypropyl)-3 dimethylhydroxyethyl ammoniumbromid)
DOSC (1,2-dioleoyl-3-succinyl-sn-glycerl cholinester)
DOGSDSO (1,2-dioleoyl-sn-glycero-3-succinyl-2hydroxyethyl disulfide ornithin),
DDAB Dimethyldioctadecylammonium bromid
DOGS ((C18)$_2$GlySper3$^+$) N,N-dioctadecylamido-glycyl-spermin (Transfectam®)
(C18)$_2$Gly$^+$ N,N-dioctadecylamido-glycin
CTAB Cetyl-trimethylammoniumbromid
CPyC Cetyl-pyridiniumchlorid
DOEPC 1,2-dioleoyl-sn-glycero-3-ethylphosphocholin oder andere 0-Alkyl-Phosphatidylcholin oder-ethanolamine, Amide aus Lysin, Arginin oder Ornithin und Phosphatidylethanolamin

Beispiele für schwach kationische Verbindungen sind:

His-Chol Histaminyl-Cholesterolhemisuccinat, Mo-Chol Morpholin-N-ethylamino-cholesterolhemisuccinat oder Histidinyl-PE.

Beispielhafte für neutrale Verbindungen sind: Cholesterol, Ceramide, Phosphatidylcholine, Phosphatidylethanolamine, Tetraetherlipide oder Diacylglycerole.

Beispielhafte schwach anionische Verbindunge sind: CHEMS Cholesterolhemisuccinat, Alkylcarbonsäuren mit 8 bis 25 C-Atomen oder Diacylglycerolhemisuccinat. Weitere schwach anionische Verbindungen sind die Amide aus Asparaginsäure, oder Glutaminsäure und PE sowie das PS und dessen Amide mit Glycin, Alanin, Glutamin, Asparagin, Serin, Cystein, Threonin, Tyrosin, Glutaminsäure, Asparaginsäure oder anderen Aminosäuren oder Aminodicarbonsäuren. Nach dem gleichen Prinzip sind auch die Ester aus Hydroxycarbonsäuren oder Hydroxydicarbonäsuren und PS schwach anionische Verbindungen.

Stark anionische Verbindungen sind beispielsweise: SDS Natriumdodecylsulfat, Cholesterolsulfat, Cholesterolphosphat, Cholesterylphosphocholin, Phosphatidylglycerole, Phosphatidsäuren, Phosphytidylinositole, Diacylglycerolphosphate, Diacylglycerolsulfate, Cetylphosphat oder Lysophospholipide.

Amphotere Verbindungen sind z.B.:

Hist-Chol Nα-Histidinyl-Cholesterolhemisuccinat, EDTA-Chol Ethylendiamintetraessigsäure-Cholesterolester, Hist-PS Nα-Histidinyl-Phosphatidylserin oder N-Alkylcarnosin.

Die erfindungsgemäßen Liposomen enthalten variable Anteile solcher membranbildender oder membranständiger Amphiphile, dass sie einen amphoteren Charakter erhalten. Das heißt, dass die Liposomen ihr Ladungsvorzeichen vollständig wechseln können. Die Menge der bei einem gegebenen pH-Wert des Mediums vorliegenden Ladungsträger eines Liposoms kann nach der folgenden Formel berechnet werden:

$$z = \Sigma ni * ((qi-1) + (10^{(pK-pH)}/(1+10^{(pK-pH)})))$$

qi absolute Ladung der einzelnen ionischen Gruppe unterhalb ihres pK (Bsp. Carboxyl =0, einfache Stickstoffbase = 1, Phosphatgruppe der zweiten Dissoziationsstufe = -1 etc.) ni Anzahl dieser Gruppen im Liposom.

Am isoelektrischen Punkt ist die Nettoladung des Liposomes 0. Durch Mischung anionischer und kationischer Anteile können Strukturen mit weitgehend wählbarem isoelektrischen Punkt erzeugt werden.

Die Strukturen können also insbesondere so konstruiert werden, dass mit fallendem pH-Wert eine wirkliche Umladung des Gesamtmoleküls von negativ auf positiv erfolgt. Eine solche Umladung ist insbesondere vorteilhaft, wenn die mit den Strukturen hergestellten Liposomen in physiologischen Zusammenhängen eingesetzt werden sollen. Nur Liposomen mit einer negativen Gesamtladung sind mit Blut- und Serumbestandteilen verträglich. Eine positive Ladung führt zu Aggregationen. Liposomen mit positiver Ladung sind aber sehr gut fusogen und können Wirkstoffe in Zellen transportieren. Eine pH-abhängige Umladung erlaubt daher die Konstruktion von serumkompatiblen, weil negativ geladenen Verbindungen, die sich nach endozytotischer Aufnahme umladen und somit erst in der Zelle fusogen werden.

In einer bevorzugten Ausführungsvariante der Erfindung weisen die amphoteren Liposomen einen isoelektrischen Punkt zwischen 5 und 7 auf.

Die Erfindung betrifft auch amphotere Liposomen, die mindestens einen amphoteren Ladungsträger umfassen, wobei der amphotere Ladungsträger einen isoelektrischen Punkt zwischen 4 und 8 aufweist.

In einer bevorzugten Ausführungsvariante weist der amphotere Ladungsträger der Liposomen einen isoelektrischen Punkt zwischen 5 und 7 auf.

Die Erfindung betrifft auch amphotere Liposomen, wobei die Liposomen mindestens einen amphoteren Ladungsträger und einen anionischen und/oder kationischen Ladungsträger umfassen.

Zweckmäßig ist es, dass in einer bevorzugten Ausführungsvariante die amphoteren Liposomen einen isoelektrischen Punkt zwischen 5 und 7 aufweisen.

In einer besonderen. Ausführungsvariante der Erfindung umfassen die erfindungsgemäßen Liposomen Phospatidylcholin, Phosphatidylethanolamin, Diacylglycerol, Cholesterol, Tetraetherlipid, Ceramid, Sphingolipid und/oder Diacylglycerol. Die Herstellung der Liposomen kann aber selbstverständlich mit vielen Lipidkombinationen entsprechend der erfindungsgemäßen Lehre ausgeführt werden. So können beispielsweise Liposomen unter Verwendung einer hohen Menge CHEMS (ca. 40%) und einer kleineren Menge DOTAP (ca. 30%) hergestellt werden. Beim pK-Wert der Carboxylgruppe des CHEMS ist die negative Ladung dieser Komponente bereits soweit zurückgedrängt, das der positive Ladungsträger in der Summe überwiegt. Eine alternative Formulierung ist die Mischung von CHEMS mit HisChol, wobei hier die stärkere Aufladung des positiven Ladungsträgers HisChol mit der Entladung des negativen CHEMS synergistisch einhergeht.

Wird die von sich aus amphotere Verbindung Hist-Chol in eine neutrale Membran, beispielsweise aus einem Phosphatidylcholin, eingebaut, so resultiert ebenfalls ein amphoteres Liposom mit einem isoelektrischen Punkt, der dem des Hist-Chol weitgehend entspricht.

Dem Fachmann ist bekannt, wie durch vielfältige Variationen der erfindungsgemäßen Lehre die wichtige Parameter anzupassen sind:

i) die Ladungsdichte der Liposomen an den Endpunkten der Umladungen durch die Menge und die pKa-Werte der verwendeten Ladungsträger,

ii) die Steilheit der Umladungskurve durch das Verhältnis der beiden Ladungsträger, durch deren absolute Mengen und durch eine ggf. synergistische Wirkung von zwei komplementären pH-sensitiven Lipiden und

iii) der Nulldurchgang des Zetapotentials durch das Verhältnis der beiden Ladungsträger wie auch durch die Lage des pK-Wertes oder der pK-Werte.

In einer weiteren Ausführungsvariante der Erfindung weisen die Liposomen eine mittlere Größe zwischen 50 und 1000 nm, bevorzugt zwischen 70 und 250 nm, besonders bevorzugt zwischen 60 und 130 nm auf. Die Herstellung der amphoteren Liposomen erfolgt nach den im Stand der Technik bekannten Methoden, also beispielsweise durch Ethanolinjektion einer Lipidlösung in wäßrige Puffer, durch Hydratisierung von trockenen Lipidfilmen oder durch Detergenzdialyse. Die Größe der Liposomen kann generell zwischen 50 nm und 10000 nm variieren. Homogene Populationen können durch Hochdruckhomogenisation oder Extrusion hergestellt werden.

In einer bevorzugten Ausführungsvariante der Erfindung umfassen die Liposomen einen Wirkstoff.

Zweckmäßig in einer bevorzugten Ausführungsvariante ist der Wirkstoff ein Protein, ein Peptid, eine DNA, eine RNA, ein antisense-Nukleotid und/oder ein Decoy-Nukleotid.

In einer weiteren bevorzugten Ausführungsvariante der Erfindung befinden sich mindestens 80% des Wirkstoffes im Innern des Liposoms.

Die Erfindung betrifft auch ein Verfahren zur Wirkstoffbeladung der Liposomen, wobei ein definierter pH-Wert zur Verkapselung benutzt wird und ein zweiter pH-Wert zur Abtrennung des nicht gebundenen Materials eingestellt wird.

Weiterhin betrifft die Erfindung auch ein Verfahren zur Wirkstoffbeladung der Liposomen, wobei die Liposomen bei einem definierten pH-Wert permeabilisiert und verschlossen werden.

Die Erfindung betrifft auch die Verwendung der Liopsomen zur Herstellung von Nanokapseln durch Abscheidung von Polymeren oder Polyelektrolyten auf der Lipidschicht. Dabei kann eine einfache oder mehrfache Abscheidung solcher Substanzen auf der Oberfläche erfolgen. Bei einer mehrfachen Abscheidung, die gegebenenfalls unter Anwesenheit von Vernetzer durchgeführt wird, entstehen liposomale Nanoakapseln, wie sie in der WO 00/28972 oder in der WO01/64330 beschrieben sind. Vorteilhaft bei der Verwendung der hier beschriebenen Substanzen ist die Tatsache, dass die elektrostatische Interaktion mit dem Polyelektrolyten unterbrochen werden kann. Es ist bekannt, dass die Wechselwirkung eines Polyelektrolyten mit Ladungsträgern der liposomalen Membran zur Entmischung von Membranbestandteilen und zur Bildung von Lipidclustern führen kann. In vielen Fällen geht diese Entmischung mit einer Permeabilisierung des Liposoms einher. Die erfindungsgemäßen Substanzen ermöglichen eine Abschaltung dieser Wechselwirkung nach dem Beschichtungsprozess. Wird der pH-Wert zu diesem Zeitpunkt erhöht, so sind die Liposomen nur noch sterisch in der Nanokapseln eingeschlossen, eine Wechselwirkung der Membran mit den Polyelektrolyten besteht dann nicht mehr. Clusterbildung der Lipide und damit verbundene Permeabilisierung der Membran können so umgangen werden.

Die Erfindung betrifft auch die Verwendung der erfindungsgemäßen Liposomen zur Verpackung und Freisetzung von Wirkstoffen. In dieser Ausführungsvariante dienen die Liposomen insbesondere der effizienten Verpackung von Wirkstoffen; beispielsweise Nukleinsäuren. Nukleinsäuren werden mit den genannten Lipiden insbesondere bei einem niedrigen pH-Wert (ca. 3 bis 6) inkubiert. Nach Bildung der Liposomen können außen anhaftende Nukleinsäuren durch den Wechsel zu einem hohen pH-Wert (ca. 7...9) abgewaschen werden.

Ein analoges Vorgehen kann für die Verpackung von Proteinen gewählt werden. Hier wird mit Vorteil ein pH im Medium eingestellt, der zwischen dem pI des Liposoms und dem des Proteins liegt. Als besonders vorteilhaft erweist es sich, wenn die beiden pI-Werte mehr als eine Einheit auseinandere liegen.

In einer weiteren Ausführungsvariante der Erfindung werden die Liposomen zur Herstellung von Freisetzungssystemen in der Diagnostik verwendet.

In einer weiteren bevorzugten Ausführungsvariante der Erfindung werden die Liposomen als Transfektionssystem verwendet, das heißt zum Einbringen von Wirkstoffen in Zellen.

In einer weiteren Ausführungsvariante der Erfindung werden die Liposomen zur gesteuerten Freisetzung ihres Inhalts durch Fusion oder Permeabilisierung der Membran verwendet. So können Liposomen aus einem allein nicht membranbildenden Lipid, etwa PE durch den Einbau von Ladungsträgern stabilisiert werden. Wird der Ladungsträger in einen neutralen ungeladenen oder zwitterionischen Zustand überführt, so erhöht sich die Permeabilität der Membran. Bekannte Liposomen nach dem Stand der Technik erlauben (PE/CHEMS, Tachibana et al.) eine solche Permeabilisierung bei niedrigen pH-Werten, wie sie unter physiologischen Bedingungen nur im Inneren von Endosomen oder bei einer Magenpassage erreicht werden. Amphotere Liposomen können nach den oben ausgeführten Maßnahmen so hergestellt werden, dass ihr Neutralpunkt bei jedem gewünschten pH-Wert zwischen 4 und 9 liegt. Unter diesen Bedingungen sind die Liposomen permeabel und können ein Cargo ins Medium abgeben.

Die liposomalen Formulierungen können jedoch unter Bedingungen geringer Permeabilität hergestellt, prozessiert und gelagert werden. In einer bevorzugten Ausführungsform der Erfindung werden Liposomen so hergestellt, dass sie unter Bedingungen eines physiologischen pH-Wertes ihre Cargo freisetzen, bei einem niedrigen pH-Wert jedoch ihr Cargo sicher einschließen. Solche Liposomen eignen sich besonders zur Herstellung von Formulierungen mit einer langsamen Freisetzungskinetik, wobei die Freisetzung erst durch den Kontakt mit Körperflüssigkeiten initiiert wird, nicht jedoch schon bei der Lagerung oder beim Transport.

Eine bevorzugte Ausführung der erfindungsgemäßen Lehre besteht daher im dem Einsatz solcher Liposomen zu therapeutischen Zwecken, insbesondere für solche Anwendungen, die ein spezifisches Targeting der Liposomen benutzen. Die geringe unspezifische Bindung ist hier Voraussetzung für einen Transport der Liposomen bis zum Zielort. Eine hohe unspezifische Bindung würde im Gegensatz dazu den Transport der Liposomen zu ihrem Zielort verhindern. Eine spezifische Bindung kann durch weitere Maßnahmen nach dem Stand der Technik erreicht werden, also durch eine Größenselektion der Liposomen oder auch die Bindung von Liganden an die liposomale Oberfläche, der an einen Zielrezeptor der Zelloberfläche bindet. Liganden können beispielsweise Antikörper oder deren Fragmente, Zuckerstoffe, Hormone, Vitamine, Peptide wie zB. das Arg-Gly-Asp (RGD), Wachstumsfaktoren, Bilirubin, oder anderen Komponenten sein.

Eine bevorzugte Ausführungsvariante der erfindungsgemäßen Lehre betrifft die Verwendung der Liposomen für therapeutische oder diagnostische Anwendungen unter in vivo-Bedingungen. Bevorzugt sind solche Liposomen, die eine geringe unspezifische Bindung und damit Fusionsneigung unter physiologischen Bedingungen zeigen, aber eine starke Bindung und hohe Fusionskompetenz unter veränderten Bedingungen aufweisen. Solche Liposomen sind amphotere Liposomen, die unter physiologischen Bedingungen eine anionische Gesamtladung des Partikels besitzen, bei einem pH<6,5 jedoch eine zunehmende kationische Aufladung zeigen. Solche pH-Werte kommen bei der Endozytose der Liposomen in Zellen vor. Solche pH-Werte kommen auch im Innern von Tumoren vor. Diese pH-Werte findet man auch in den äußeren Schichten der Haut. Niedrige pH-Werte können auch ex vivo bei der Perfundierung eines Organs für einen gewissen Zeitraum eingestellt werden. Eine hohe Bindungsstärke und Fusionskompetenz ist daher auf solche Liposomen beschränkt, die bereits von Zellen oder speziellen Geweben aufgenommen wurden. Bindungsstärke und zunehmende Fusionskompetenz unterstützen die Verschmelzung der liposomalen Membran mit der Zellmembran. Dieses Ereignis führt zu einer direkten Freisetzung des Cargos in das Zellinnere, ohne lytische Komponenten des Endosoms freizusetzen und damit das Cargo oder Zellbestandteile zu gefährden.

Zweckmäßig ist weiterhin die Verwendung der Liposomen als Depotformulierung und/oder als zirkulierendes Depot. Mit Vorteil können die Liposomen auch bei intravenöser oder peritonealer Applikation verwendet werden. In einer besonders bevorzugten Ausführungsvariante der Erfindung werden die Liposomen als Vektor zur Transfektion von Zellen in vivo, in vitro und ex vivo eingesetzt.

Die erfindungsgemäßen Liposomen weisen mehrere Vorteile auf. Kationisch aufladbare Liposomen aus 40% HisChol und PC binden auch unter Bedingungen eines neutralen pH-Wertes Nukleinsäuren, wie z.B. DNA an ihrer Membran. Überraschenderweise wird diese Bindung vollständig unterdrückt, wenn die oben angegebenen Liposomen unter zusätzlicher Verwendung von 5% PG hergestellt werden und dann amphotere Eigenschaften haben. Die Bindung von Nukleinsäuren an die Membran ist jedoch durch Verringerung des pH-Wertes wieder herstellbar. Liposomen nach der erfindungsgemäßen Lehre sind daher sehr gut zur pH-abhängigen Bindung von Nukleinsäuren geeignet.

Es wurde weiterhin überraschend gefunden, dass auch eine Reihe von Proteinen sich in der für die Nukleinsäuren beschriebenen Art verhalten. So binden Antikörper nicht bei neutralen pH-Wert, wohl aber unter leicht sauren Bedingungen effektiv an die Membran der erfindungsgemäßen Liposomen. Ein solches Verhalten kann weder bei pH-sensitiven Liposomen aus einem Neutrallipid und CHEMS noch bei solchen aus einem Neutrallipid und HisChol beobachtet werden. Es ist daher eine besondere Eigenschaft der amphoteren Liposomen. Es überraschenderweise auch gefunden, dass Liposomen gemäß der vorliegenden Erfindung im Gegensatz zu den bekannten konstitutiv kationischen Liposomen serumkompatibel sind. Eine zweckmäßige Ausführung der erfindungsgemäßen Lehre besteht daher beim Einsatz solcher Liposomen zu therapeutischen Zwecken. Ein Vorteil der Liposomen ist, dass sie eine wesentlich geringere unspezifische Bindung an Zellen aufweisen, als dies bei bekannten konstitutiv kationischen Liposomen der Fall ist.

Überraschend ist auch, dass die Fusionskompetenz der erfindungsgemäßen Liposomen vom pH-Wert des Mediums abhängig ist. Die Fusionskompetenz gegenüber biologischen Membranen von Zellen wird durch die Wahl des Lipids, aber auch durch die Aufladung der Liposomen bestimmt. Der eigentlichen Fusion geht für gewöhnlich ein Bindungsschritt voraus. Eine starke Bindung der Liposomen an Zellmembranen aber nicht immer wünschenswert, sondern soll wie oben beschrieben nur unter kontrollierten Bedingungen in bestimmten Zellen oder Geweben erfolgen.

Die Liposomen können daher zur Konstruktion von liposomalen Vektoren für den Transport von Wirkstoffen in Zellen genutzt werden. Als Wirkstoffe kommen alle Stoffe in Frage, die nicht mizellbildend sind. Besonders geeignete Wirkstoffe sind wasserlösliche Stoffe. Das sind viele Proteine und Peptide, insbesondere Antikörper oder Enzyme oder Antigene, alle Nukleinsäuren, unabhängig von ihrem Molekulargewicht und ihrer Abstammung von RNA oder DNA. Das sind aber auch andere biologische Makromoleküle wie etwa komplexe Zucker, Naturstoffe und weitere Verbindungen. Das sind ebenfalls niedermolekulare Wirkstoffe synthetischen oder natürlichen Ursprungs, die sonst nicht die Zellmembran als Barriere durchdringen können. Solche Stoffe können dann mit Hilfe der Vektoren in das Innere von Zellen transportiert werden und Wirkungen auslösen, die ohne diesen Transport nicht möglich wären.

Mit Hilfe der erfinderischen Lehre können somit Liposomen hergestellt werden, deren Fusions- und Bindungseigenschaften sich bei verschiedenen pH-Werten unterscheiden. Es können daher auf diesem Wege serumkompatible Liposomen hergestellt werden, die mit einer großen Menge von Wirkstoffen beladen sind und diese in das Innere von Zellen transportieren. Es ist dem Fachmann möglich, Elemente der erfindungsgemäßen Lehre miteinander zu kombinieren und damit Liposomen herzustellen, die optimal für einen bestimmten Zweck geeignet sind.

Die Erfindung soll im folgenden anhand von Beispielen näher erläutert werden, ohne dass die Erfindung auf diese Beispiele zu beschränken ist.

**Beispiel 1**

**Herstellung und Ladungseigenschaften amphoterer Liposomen mit positiv aufladbarem und konstant negativ geladenem Ladungsträger**

5 mg His-Chol und 7.8 mg POPC und 2 mg DPPG werden in 4 mL Chloroform/Methanol (1:1 v/v) gelöst und im Rotationsverdampfer vollständig getrocknet. Der Lipidfilm wird mit 4.3 mL des entsprechenden Puffers (10 mM KAc, 10 mM HEPES, 150 mM NaCl, pH 7,5 in einer Lipidkonzentration von 5 mM durch 5 min Ultraschallbehandlung hydratisiert. Abschließend wird die Suspension eingefroren und nach dem Auftauen mehrfach extrudiert (Avestin LiposoFast, Polycarbonatfilter 200nm Porenweite). Zur Messung des Zetapotentials wird eine Endkonzentration der Liposomen von 0,2 mM eingestellt. Zur Verdünnung wird das oben genannte Puffersystem bei einem pH von 7,5 bzw. 4,2 benutzt. Die gemessenen Zetapotentiale liegen bei -18mV (pH7.5) bzw. bei +35mV (pH4.2).

**Beispiel 2**

**Herstellung und Ladungseigenschaften amphoterer Liposomen mit konstant positivem und veränderlich negativem Ladungsträger**

POPC, DOTAP und CHEMS werden in den unten angegebenen molaren Verhältnissen in 4 mL Chloroform/Methanol (1:1 v/v) gelöst und im Rotationsverdampfer vollständig getrocknet. Der Lipidfilm wird mit 4.3 mL des entsprechenden Puffers (10 mM KAc, 10 mM HEPES, 150 mM NaCl, pH 7,5) in einer Gesamtlipidkonzentration von 5 mM durch 5 min Ultraschallbehandlung hydratisiert. Abschließend wird die Suspension eingefroren und nach dem Auftauen mehrfach extrudiert (Avestin LiposoFast, Polycarbonatfilter 200nm Porenweite). Die untenstehende Tabelle zeigt die Zetapotenziale in Abhängigkeit vom pH.

Zusammensetzung der Liposomen in mol-%

| Liposom 1 | POPC 50 | DOTAP 40 | Chems 10 |
| Liposom 2 | POPC 50 | DOTAP 30 | Chems 20 |
| Liposom 3 | POPC 50 | DOTAP 25 | Chems 25 |
| Liposom 4 | POPC 50 | DOTAP 20 | Chems 30 |
| Liposom 5 | POPC 50 | DOTAP 40 | Chems 10 |

Tabelle 1. Zetapotenziale in mV

| pH-Wert | Liposom1 | Liposom2 | Liposom3 | Liposom4 | Liposom5 |
|---|---|---|---|---|---|
| 4 | 44,2 | 38,4 | 34,7 | 31,7 | 16,2 |
| 5 | 39,9 | 25,6 | 27,2 | 22,1 | 3,3 |
| 6 | 37 | 21,4 | 16,4 | 2,5 | -7,3 |
| 7,5 | 29,2 | 1,8 | -7,9 | -18,9 | -34,6 |

Durch geeignete Zusammensetzung ist die Höhe des Zetapotenzials und dessen Steilheit in weiten Grenzen wählbar.

**Beispiel 3**

**Herstellung und Ladungseigenschaften amphoterer Liposomen mit kompletter Schaltbarkeit in einer Verbindung**

5 mg Hist-Chol und 9.8 mg POPC werden in 4 mL Chloroform/Methanol (1:1 v/v) gelöst und im Rotationsverdampfer vollständig getrocknet. Der Lipidfilm wird mit 4.3 mL des entsprechenden Puffers (10 mM Kac, 10 mM HEPES, 150 mM NaCl, pH 7,5 in einer Lipidkonzentration von 5 mM durch 5 min Ultraschallbehandlung hydratisiert. Abschließend wird die Suspension eingefroren und nach dem Auftauen mehrfach extrudiert (Avestin LiposoFast, Polycarbonatfilter 200nm Porenweite). Der Verlauf des Zetapotentials bei verschiedenen pH-Werten und Ionenstärken ist in der untenstehenden Tabelle dargestellt (Tabelle 2).

Tabelle 2

| pH-Wert | ohne Salz | 100 mM NaCl |
|---|---|---|
| 4 | 45,6 | 20,2 |
| 5 | 26,9 | 2,2 |
| 6 | -4,1 | -5,2 |
| 7 | -31,4 | -15,3 |
| 8 | -45,7 | -25,4 |

**Beispiel 4**

**Serumaggregation**

Lipidfilme werden wie in Beispiel 1 hergestellt. Als Vergleichprobe dient eine Lipidmischung, die kein DPPG enthält. Die Lipidfilme werden in Puffer (10mM Phosphat, 150mM NaCl, pH7.4) hydratisiert und wie oben extrudiert. Humanes Serum wird mit einer gleichen Menge Puffer (10mM Phophat, 150mM NaCl, pH 7.4) verdünnt, partikuläre Bestandteile und Fett werden durch Zentrifugation (20min, 13.00rpm, 4°C) entfernt, das klare Serum wird mit einem Filter der Porenweite 0.2μm steril filtriert.

Die oben präparierten Liposomen werden in einer Konzentration von 1mM zum Serum gegeben und für 15min bei 37°C inkubiert. Nach der Inkubation ist die Suspension der DPPG-haltigen Liposomen gleichmäßig trüb, ohne das eine Flockung beobachtet werden kann. Der Durchmesser der Liposomen wird mittels dynamischer Lichtstreuung bestimmt und ist um weniger als 10% gegenüber der Ausgangsprobe verändert. Die Suspension der DPPG-freien Liposomen zeigt deutliche Flockung.

**Beispiel 5**

**Serumstabilität der Membran**

Neben der Serumaggregation wurde auch das Austreten eines Wirkstoffes (Carboxyfluorescein, CF) in Gegenwart von Humanserum untersucht. Dazu wurden POPC/DOTAP/CHEMS Liposomen unterschiedlicher Zusammensetzung nach Beispiel 2 hergestellt:
POPC 100% (als Kontrolle), POPC/DOTAP/CHEMS 60:30:10, 60:20:20 und 60:10:30 (Angaben in Mol%). Nicht eingeschlossenes CF wurde durch Gelfiltration abgetrennt. Zur Messung wurden die Liposomen auf 0.1 mM in Serum verdünnt und bei 37°C inkubiert. Zu bestimmten Zeitpunkten wurde eine Probe von 30 μl entnommen und mit 100 mM TRIS Puffer, pH 8.2 auf 300 μl verdünnt und die Fluoreszenz gemessen. Die 100% Werte wurden durch Auflösen der Liposomen mit 10 μl Triton X-100 (10% in Wasser)gewonnen. Der Zeitverlauf des eingeschlossenen CF ist in der untenstehenden Tabelle dargestellt.
Die Liposomen verlieren nur wenig CF in Serum über den gemessenen Zeitraum von 4 h. POPC/DOTAP/ CHEMS 60:30:10 und 60:20:20 besitzen nach 4 h noch circa 75%, POPC und POPC/DOTAP/CHEMS 60:10:30 sogar an die 100% ihres ursprünglichen CF-Gehalts (siehe Tabelle 3).

**Tabelle 3.**

| Zeit in min | POPC | POPC/DOTAP/CHEMS 60: 30:10 | POPC/DOTAP/CHEMS 60: 20:20 | POPC/DOTAP/CHEMS 60: 10:30 |
|---|---|---|---|---|
| 0 | 100% | 100% | 100% | 100% |
| 15 | 91% | 84% | 95% | 107% |
| 60 | 94% | 81% | 87% | 110% |
| 120 | 96% | 80% | 76% | 105% |
| 240 | 96% | 80% | 77% | 107% |

**Beispiel 6**

**Bindung von DNA**

Liposomen mit folgenden Zusammensetzungen werden wie in Beispiel 1 hergestellt: (alle Angaben in Mol%)

| | | | |
|---|---|---|---|
| A: | 60 POPC | 40 HisChol | |
| B: | 55 POPC | 40 HisChol | 5 CHEMS |
| C: | 60 POPC | 20 HisChol | 20 CHEMS |

Die Liposomen werden in einer Konzentration von 0.2mM in Puffer (10 mM Kaliumacetat, 10 mM HEPES, pH 4.2 bzw. 7.5) suspendiert. 45 μl einer DNA-Lösung (1mg DNA (Hering sperm, SIGMA D3159)in 1 ml Wasser) werden zu jeweils 1 ml der unterschiedlichen Liposomenproben gegeben und schnell gemischt. Nach 15min Inkubation wird die Probe mit 6 ml des entsprechenden Puffers aufgefüllt und das Zetapotential der Liposomen vermessen (Tabelle 4).

**Tabelle 4.**

| Lipid | pH 4.2 | | pH 7.5 | |
|---|---|---|---|---|
| | -DNA | +DNA | -DNA | +DNA |
| A | + 47.6 | - 32.0 | + 2.4 | - 44.4 |
| B | + 47.8 | - 28.1 | + 0.1 | - 38.4 |
| C | + 34.0 | - 28.6 | - 10.1 | - 24.7 |

Unter den Bedingungen eines Überschusses kationischer Ladungen (pH 4.2) findet eine starke Umladung der Partikel statt. Beim neutralen pH von 7.5 kann das CHEMS in hoher Konzentration (Liposom C)die Ladung des HisCHol überkompensieren, die Partikel haben ein negatives Zetapotential. An solche Partikel binden nur noch geringe Mengen DNA.

**Beispiel 7**

**Bindung und Ablösung von DNA**

Liposomen der Zusammensetzungen POPC/DOTAP/CHEMS 60:15:25 und POPC/DCChol/CHEMS 60:15:25 (alle Angaben in Mol%)wurden nach Beispiel 2 hergestellt. Die Bindung von DNA wurde nach obigem Beispiel bei pH4,2 durchgeführt und die Zetapotentiale bestimmt. Anschließend wurden die Proben auf einen pH von 7,5 eingestellt und wiederum das Zetapotential gemessen.

| Mischung | | Zeta [mV] |
|---|---|---|
| a) | POPC/DCChol/CHEMS 60:15:25 (pH 4,2) (Aggregate) | -43,5 |
| b) | POPC/DOTAP/CHEMS 60:15:25 (pH 4,2) | -43,7 |
| c) | POPC/DCChol/CHEMS 60:15:25 (pH 7,5) | -18,5 |
| d) | POPC/DOTAP/CHEMS 60:15:25 (pH 7,5) | -14,5 |

In Gegenwart von DNA wird bei niedrigem pH ein negatives Zetapotential gemessen, die ursprünglichen Partikel waren jedoch positiv geladen. Nach dem Wechsel zum neutral-pH verringert sich diese durch DNA bedingte Aufladung. Die Zetapotentiale nähern sich dem der unbehandelten Liposomen (-11mV bei pH 7,5)

**Beispiel 8**

**DNA-Einschluß und Ablösung nicht verkapselten Materials**

Zwei Liposomenformulierungen der Zusammensetzung POPC60/DOTAP15/CHEMS25 bzw. POPC85/DOTAP15 werden als trockene Lipidfilme wie oben beschrieben hergestellt. Die Gesamtmenge des Lipids betrug jeweils 4μMol. Zur Hydratisierung wurde Herings-DNA in 10mM Kac, 10mM HEPES und 100mM NaCl pH4,0 gelöst. 4mg der DNA wurde direkt zu den Lipidfilmen gegeben. Die entstandenen Liposomen wurden mehrfach eingefroren und getaut und anschließend durch ein 200nm -Filter extrudiert.

Je 500μl der Partikel wurden mit 2,5 ml einer Sucroselösung gemischt (0,8M Sucrose in Puffer wie oben, pH-Wert 4,0 oder 7,5) und mit 1,5ml einer 0,5M-Sucroselösung sowie 0,5ml des Puffers überschichtet.

Liposomen wurden dann von nicht gebundener DNA durch Flotation getrennt. Die Liposomen wurden nach der Flotation von der Grenzfläche Puffer / 0,5M Sucrose abgenommen.

Die Bestimmung der gebundenen DNA-Menge erfolgt durch Interkalation von Propidiumiodid, für die Bestimmung der Lipidmenge wurde der Stewart-Assay verwendet. Im Stewart-Assay spricht nur das verwendete PC an, die anderen Lipide wurden anhand dieses Wertes berechnet. Die Ergebnisse sind in der untenstehenden Tabelle dargestellt (Tabelle 5).

**Tabelle 5.**

| Liposom | pH 4, 0 | pH 7,5 |
|---|---|---|
| POPC/DOTAP/CHEMS 60/15/25 | 2μg DNA/μg DOTAP | 1,2μg DNA/μg DOTAP |
| POPC/DOTAP 85/15 | 2,3μg DNA/μg DOTAP | 2,3μg DNA/μg DOTAP |

Mit den amphoteren Liposomen flotiert nach dem pH-Wechsel auf 7,5 nur noch etwa die Hälfte der gebundenen DNA nach oben. Dieses Material ist das wirklich eingeschlossene Material. Analoge Ergebnisse wurden bei einem Verdau mit DNAse erhalten.

Von konstitutiv kationischen Liposomen lässt sich DNA durch pH-Wechsel und auch durch eine zusätzliche Erhöhung der Ionenstärke nicht wieder ablösen und verbleibt immer an der Aussenseite.

**Beispiel 9**

**Fusionseigenschaften**

Liposomen mit folgenden Zusammensetzungen werden wie in Beispiel 1 hergestellt (alle Angaben in Mol-%):

|   |   |   |   |
|---|---|---|---|
| A) | POPC 60 | HisChol 40 | |
| B) | POPC 55 | HisChol 40 | CHEMS 5 |
| X) | POPC 100 | | |
| Y) | POPC 60 | DPPG 40 | |

Die fakulativ kationischen Liposomen A oder B werden mit den neutralen Liposomen X oder den anionischen Liposomen Y im Puffer (10mM HEPES, 10mM Kaliumacetat, pH4.2 bzw. 7.5)inkubiert. Die eventuelle Fusion von Liposomen wird mittels Größenmessung durch dynamische Lichtstreuung analysiert (Tabelle 6).

**Tabelle 6.**

| Liposom 1 | X | X | Y | Y |
|---|---|---|---|---|
| Liposom 2 | A | B | A | B |
| pH 4.2 | 181,6 nm | 191,9 nm | 1689,3 nm | 2373,2 nm |
| pH 7.5 | 191,8 nm | 202,4 nm | 250,0 nm | 206,3 nm |

Die Ausgangsgrößen der Liposomen betrugen bei pH 4.2 161,8 nm und 165,9 nm bei pH 7.5

A) 183 , 2nm
X) 199,2nm
Y) 183,2nm

Die Größe der komplementär geladenen Paare (YA und YB) unterscheidet sich deutlich von der Größe der Mischsuspensionen mit dem Neutralliposom X. Das Ausmaß der Wechselwirkung ist durch das Maß der Aufladung der fakultativ kationischen Liposomen bestimmt. Eine Fusion zu größeren Einheiten ist nicht von dem fusogenen Lipid PE abhängig.

**Beispiel 10**

**Permeabilität gegenüber Makromolekülen**

13.75 $\mu$mol DOPE, 2.5 $\mu$mol CHEMS und 10 $\mu$mol HisChol werden in Isopropanol gelöst und das Lösungsmittel wird unter Vakuum abgezogen. Zu dem getrockneten Lipidfilm gibt man 2.5ml einer Lösung von Proteinase K in Puffer (1mg/ml Proteinase K, 10mM Kaliumacetat, 10mM HEPES, 150mM NaCl, pH4.2). Nach der Hydratisierung des Films werden die gebildeten Liposomen durch eine 400nm-Membran extrudiert. Nicht eingeschlossene Proteinase wird durch Flotation der Liposomen im Sucrosegradienten abgetrennt. Die so hergestellten Liposomen werden mit 7.5ml Puffer bei pH4.2 und pH7.2 inkubiert (Puffer wie oben, Ausgangs-pH 4.2 und 8.0). Nach der Inkubation wird freigesetzte Proteinase K durch Ultrafiltration mit einer 0.1 $\mu$m-Membran abgetrennt. Die im Filter verbleibenden Liposomen werden dann mit 7.5ml einer Lösung von Triton X-100 in Puffer (wie oben, pH 8.0) behandelt.

Alle Filtrate werden auf die Anwesenheit von Proteinase K getestet. Dazu wird eine Lösung von Azocasein (6 mg/ml Azocasein in 1 M Harnstoff, 200 mM Tris-Sulfat pH 8.5) verwendet. 500$\mu$l dieser Lösung werden mit 100$\mu$l Filtrat oder Puffer gemischt und für 30min bei 37°C inkubiert. Die Reaktion wird durch Zugabe von 10% Trichloressigsäure gestoppt. Präzipitierte Proteine werden durch Zentrifugation abgetrennt. Die Färbung im Überstand wird bei 390nm gemessen (Tabelle 7).

**Tabelle 7.**

| pH Inkubation | Triton X100 | Absorption bei 390nm - Blank |
|---|---|---|
| 4.2 | - | 0,0192 |
| 4.2 | + | 0,2345 |

(fortgesetzt)

| pH Inkubation | Triton X100 | Absorption bei 390nm - Blank |
|---|---|---|
| 7.2 | - | 0,2210 |
| 7.2 | + | 0,0307 |

Erfolgt die Inkubation der Liposomen bei einem pH-Wert von 4.2, so wird keine oder nur sehr wenig Proteinase K freigesetzt. Erst die Auflösung der Liposomen mit Triton X100 führt zur Freisetzung des Enzyms. Wenn die Liposomen bei einem pH-Wert von 7.2 inkubiert werden, so wird bereits ohne Zugabe von Triton der Großteil des Enzyms freigesetzt und findet isch im ersten Filtrat. Die Zugabe von Triton kann dann kaum noch weiteres Enzym aus den Liposomen herauslösen.

**Beispiel 11**

**Proteinbindung**

Liposomen der Zusammensetzung POPC50/ DOTAP10/ CHEMS40 (alle Angaben in mol-% werden wie in den vorhergehenden Beispielen hergestellt. Zur Hydratisierung der Lipidfilme wird eine Lösung von 0,26 mg/ml Lysozym in Puffer (10 mM MES pH 5,0 oder pH 6,0 bzw. 10 mM HEPES pH 7,0 oder pH8,0) verwendet.Alle Proben werden nach der Hydratisierung mehrfach eingefroren und getaut. Anschließend werden die Liposomen mittels Ultraschall homogenisiert und durch ein 200nm-Filter extrudiert.

Die so hergestellte Liposomensuspension werden durch Zugabe von Essigsäure auf einen pH-Wert von 4,0 eingestellt. Anschließend werden die Liposomen von nicht eingebautem Protein durch Flotation getrennt. In der untenstehenden Tabelle ist der Anteil des eingeschlossenen Proteins wiedergegeben (Tabelle 8).

**Tabelle 8.**

| pH-Wert beim Einschluss | % eingeschlossenes Material |
|---|---|
| 5,0 | 4 |
| 6,0 | 21 |
| 7,0 | 75 |
| 8,0 | 80 |

Liposomen der verwendeten Zusammensetzung zeigen einen pI von 5, das Lysozym ist ein basisches Protein mit einem pI von 11,35 . Im pH-Bereich zwischen 6 und 8 sind daher beide Partner entgegengesetzt geladen. Durch die elektrostatische Anziehung wird ein effizienter Einschluß in die Liposomen bewirkt. Nicht verkapseltes Protein wurde einem pH von 4 entfernt. Bei diesem pH wird die Wechselwirkung zwischen den Partnern aufgehoben.

**Beispiel 12**

**Transfektion in Zellen**

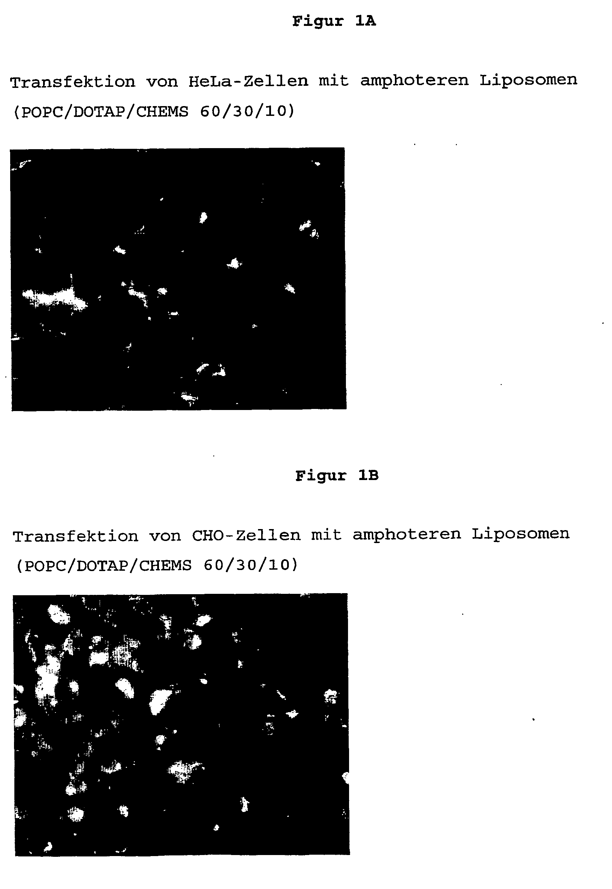

HeLa-Zellen oder CHO-Zellen ($3*10^5$) wurden in jede Kavität einer 6-well Titerplatte ausplattiert und für drei Tage kultiviert. Liposomen (POPC/DOTAP/CHEMS 60/30/10) wurden in Gegenwart von fluoreszensmarkiertem Dextran (TRITC-Dextran, 10mg/ml im Hydratisierungspuffer) hergestellt.Nicht eingebautes TRITC-Dextran wurde durch Gelfiltration entfernt. Die so hergestellten Liposomen wurden zu den Zellen gegeben und für 6h bei 37°C inkubiert. Anschließend wurden die Zellen zweimal mit Puffer gewaschen. Die Aufnahme des Dextrans wurde im mikroskopischen Bild verfolgt. Die Ergebnisse sind in der Figur 1 dargestellt.

**Beispiel 13**

**Ligandenbindung und Transfektion**

Liposomen der Zusammensetzung POPC/DOTAP/Chems/N-glutaryl-DPPE (50:10:30:10 (mol%)) werden nach Beispiel 2 hergestellt, dabei werden sie mit einer Lösung von 3mg/ml TRITC-Dextran (Mw ca. 4400)in Hepes 10 mM, 150 mM NaCl, pH 7,5 hydratisiert. Nichteingeschlossenes TRITC-Dextran wird durch Gelfiltration über eine Sephadex G-75 Säule abgetrennt. Die Bindung des cyclischen Peptides RCDCRGDCFC an die liposomale Oberfläche wurde durch Aktivierung des N-glutaryl-DPPEs mit EDC (1-Ethyl-3-(3-dimethylaminopropyl carbodiimid)erreicht (3.5 mg EDC zu 400 $\mu$l Liposomensuspension) und anschließendes Rühren im Dunkeln über 5 h. Dann wurde das RGD-Peptid (250 $\mu$g in 150 $\mu$l Puffer) zugegeben und über Nacht gerührt. Die Liposomen wurden durch Gelfiltration vom nichtgebundenen Peptid abgetrennt.

Humane Endothelzellen (HUVEC)wurden in Spezialmedium kultiviert. Die mit Ligand modifizierten Liposomen und Kontrollliposomen ohne RGD-Ligand werden als 0.5 mM Suspension auf die Zellen gegeben. Nach 2 Stunden werden die Liposomen abgenommen und die Zellkammern 3 mal mit PBS-Puffer gespült und unter dem Fluoreszenzmikroskop betrachtet. Zellen, die mit RGD-Liposomen behandelt wurden zeigten eine erheblich höhere rote TRITC- Fluoreszenz als die Kontrollliposomen.

**Beispiel 14**

**Pharmakokinetik** (Blutspiegel und Organverteilung) von pHschaltbaren Liposomen

Je 500 $\mu$L Liposomen aus POPC/Chol (60:40), POPC/Hist-Chol/Chol (60:20:20) und POPC/DOTAP/Chems (60:10:30) wurden männlichen Wistar-Ratten per Injektion in die Schwanzvene verabreicht.

50 mM Liposomen-Suspensionen wurden hergestellt durch Hydratisieren eines Lipidfilms der entsprechenden Formulierung (Addition von 0,03 mol% [14]C-DPPC) mit 2 mL einer Lösung von 1 mg [3]H-Inulin in HEPES 10 mM, NaCL 150 mM, pH 7.5). Nach 3 Einfrier/Auftau-zyklen wurden die Suspensionen durch eine 400 nm-Membran mehrfach extrudiert (LiposoFast, Avestin). Abtrennung von nichteingeschlossenem [3]H-Inulin erfolgte durch Gelfitration über eine G-75 Sephadex-Säule und anschließende Konzentrierung über CENTRIPREP (Millipore) Zentrifugationseinheiten. 4 Versuchstieren je Formulierung wurden 0.5 mL Liposomensuspension verabreicht und Blutproben nach 5 min, 15 min, 60 min, 3 h, 12 h, 24 h genommen. Die Radioaktivität der Membranfraktion und des löslichen Cargos wurden per Szintillation vermessen und ergaben folgende Werte:

Eliminationshalbwertszeiten aus dem Blut:

| | |
|---|---|
| POPC/Chol | größer 120 min |
| POPC/DOTAP/Chems | größer 120 min |
| POPC/Hist-Chol | größer 120 min |

Mit ihrer relativ langen Halbwertszeit im Blut erfüllen die erfindungsgemäßen Liposomen die Grundvoraussetzungen für ein Vektorsystem. Sie sind nicht akut toxisch und werden nicht sofort vom retikuloendothelialen System aufgenommen. Das Verhältnis der 3 [H] und der 14 [C]-Radioaktivität der Blutproben war bis zum Ende des Experiments konstant. Es findet daher in keinem Fall eine Freisetzung des Cargos durch Complementlyse statt.

**Patentansprüche**

1. Amphotere Liposomen, **dadurch gekennzeichnet, dass** die Liposomen mindestens einen positiven und mindestens einen davon verschiedenen negativen Ladungsträger umfassen, wobei die Liposomen einen isoelektrischen Punkt zwischen 4 und 8 aufweisen und die Liposomen ein neutrales Lipid umfassen, ausgewählt aus der Gruppe bestehend aus Phospatidylcholin, Phosphatidylethanolamin, Cholesterol, Tetraetherlipid, Ceramid, Sphingolipid und/oder Diacylglycerol.

2. Amphotere Liposomen nach Anspruch 1, **dadurch gekennzeichnet, dass** die Liposomen einen isoelektrischen Punkt zwischen 5 und 7 aufweisen.

3. Amphotere Liposomen, **dadurch gekennzeichnet, dass** die Liposomen mindestens einen amphoteren Ladungsträger umfassen, wobei der amphotere Ladungsträger einen isoelektrischen Punkt zwischen 4 und 8 aufweist und die Liposomen ein neutrales Lipid umfassen, ausgewählt aus der Gruppe bestehend aus Phospatidylcholin, Phosphatidylethanolamin, Cholesterol, Tetraetherlipid, Ceramid, Sphingolipid und/oder Diacylglycerol.

4. Amphotere Liposomen nach dem vorhergehenden Anspruch, **dadurch gekennzeichnet, dass** der amphotere Ladungsträger einen isoelektrischen Punkt zwischen 5 und 7 aufweist.

5. Amphotere Liposomen, **dadurch gekennzeichnet, dass** die Liposomen mindestens einen amphoteren Ladungsträger mit einem isoelektrischen Punkt zwischen 4 und 8 und einen anionischen und/oder kationischen Ladungsträger umfassen, wobei die Liposomen ein neutrales Lipid umfassen, ausgewählt aus der Gruppe bestehend aus Phospatidylcholin, Phosphatidylethanolamin, Cholesterol, Tetraetherlipid, Ceramid, Sphingolipid und/oder Diacylglcerol.

6. Amphotere Liposomen nach Anspruch 5, **dadurch gekennzeichnet, dass** die Liposomen einen isoelektrischen Punkt zwischen 5 und 7 aufweisen.

7. Amphotere Liposomen nach einem der vorhergehenden Ansprüche, **dadurch gekennzeichnet, dass** die Liposomen eine mittlere Größe zwischen 50 und 1000 nm, bevorzugt zwischen 70 und 250 nm, besonders bevorzugt zwischen 60 und 130 nm aufweisen.

8. Amphotere Liposomen nach einem der vorhergehenden Ansprüche, **dadurch gekennzeichnet, dass** die Liposomen einen Wirkstoff umfassen.

9. Amphotere Liposomen nach dem vorhergehenden Anspruch, **dadurch gekennzeichnet, dass** der Wirkstoff ein Protein, ein Peptid, eine DNA, eine RNA, ein antisense-Nukleotid und/oder ein Decoy-Nukleotid ist.

10. Amphotere Liposomen nach einem der vorhergehenden Ansprüche, **dadurch gekennzeichnet, dass** sich mindestens 80% des Wirkstoffes im Innern des Liposoms befinden.

11. Verfahren zur Wirkstoffbeladung von Liposomen gemäß der Ansprüche 1 bis 10, **dadurch gekennzeichnet, dass** ein definierter pH-Wert zur Verkapselung benutzt wird und ein zweiter pH-Wert zur Abtrennung des nicht gebundenen Materials eingestellt wird.

12. Verfahren zur Wirkstoffbeladung von Liposomen gemäß der Ansprüche 1 bis 10, **dadurch gekennzeichnet, dass** die Liposomen bei einem definierten pH-Wert permeabilisiert und verschlossen werden.

13. Verwendung von Liposomen nach einem der Ansprüche 1 bis 10 zur Herstellung von Nanokapseln.

14. Verwendung von Liposomen nach einem der Ansprüche 1 bis 10 zur Herstellung von Freisetzungssystemen in der Diagnostik.

15. Verwendung von Liposomen nach einem der Ansprüche 1 bis 10 zur Herstellung von pharmazeutischen Präparaten zum Transport und/oder zur Freisetzung von Wirkstoffen.

16. Verwendung von Liposomen nach einem der Ansprüche 1 bis 10 zur Herstellung von pharmazeutischen Präparaten als Depotformulierung und/oder als zirkulierendes Depot.

17. Verwendung von Liposomen nach einem der Ansprüche 1 bis 10 zur Herstellung eines pharmazeutischen Präparates zur intravenösen oder peritonealen Applikation.

18. Verwendung von Liposomen nach einem der Ansprüche 1 bis 10 zur Herstellung von pharmazeutischen Präparaten als Vektor zur Transfektion von Zellen in vivo und ex vivo.

19. Verwendung von Liposomen nach einem der Ansprüche 1 bis 10 zur Herstellung von Vektoren zur Transfektion von Zellen in vitro.

**Claims**

1. Amphoteric liposomes, **characterized in that** the liposomes comprise at least one positive charge carrier and at least one negative charge carrier, which is different from the positive charge carrier, the liposomes having an isoelectric point of between 4 and 8, and the liposomes comprise a neutral lipid, selected from the group consisting of phosphatidyl choline, phosphatidyl ethanolamine, cholesterol, tetraether lipid, ceramide, sphingolipid and/or diacyl glycerol.

2. Amphoteric liposomes of claims 1, **characterized in that** the liposomes have an isoelectric point of between 5 and 7.

3. Amphoteric liposomes, **characterized in that** the liposomes comprise at least one amphoteric charge carrier, the amphoteric charge carrier having an isoelectric point of between 4 and 8, and wherein the liposomes comprise a neutral lipid, selected from the group consisting of phosphatidyl choline, phosphatidyl ethanolamine, cholesterol, tetraether lipid, ceramide, sphingolipid and/or diacyl glycerol.

4. Amphoteric liposomes of the preceding claim, **characterized in that** the amphoteric charge carrier has an isoelectric point of between 5 and 7.

5. Amphoteric liposomes, **characterized in that** the liposomes comprise at least one amphoteric charge carrier having an isoelectric point of between 4 and 8 and one anionic and/or cationic charge carrier, whereby the liposomes comprise a neutral lipid, selected from the group consisting of phosphatidyl choline, phosphatidyl ethanolamine, cholesterol, tetraether lipid, ceramide, sphingolipid and/or diacyl glycerol.

6. Amphoteric liposomes of claim 5, **characterized in that** the liposomes have an isoelectric point of between 5 and 7.

7. Amphoteric liposomes of one of the preceding claims, **characterized in that** the liposomes have an average size of between 50 and 1000 nm, preferably between 70 and 250 nm and particularly between 60 and 130 nm.

8. Amphoteric liposomes of one of the preceding claims, **characterized in that** the liposomes comprise an active ingredient.

9. Amphoteric liposomes of the preceding claim, **characterized in that** the active ingredient is a protein, a peptide, a DNA, an RNA, antisense nucleotide and/or a decoy nucleotide.

10. Amphoteric liposomes of one of the preceding claims, **characterized in that** at least 80 percent of the active ingredient is in the interior of the liposome.

11. Method for charging liposomes with active ingredients of claims 1 to 10, **characterized in that** a defined pH is used for the encapsulation and a second pH is used for separating the material, which has not been bound.

12. Method for charging liposomes with active ingredient of claims 1 to 10, **characterized in that** the liposomes are permeabilized and closed off at a defined pH.

13. Use of liposomes of one of the claims 1 to 10 for producing nanocapsules.

14. Use of liposomes of one of the claims 1 to 10 for producing release systems in diagnostics.

15. Use of liposomes of one of the claims 1 to 10 for producing of pharmaceutical compositions for transporting and/or releasing active ingredients.

16. Use of liposomes of one or claims 1 to 10 for producing of pharmaceutical compositions as a sustained-release formulation and/or as a circulating depot.

17. Use of liposomes of one of the claims 1 to 10 for producing of pharmaceutical compositions for for intravenous or peritoneal application.

18. Use of liposomes of one of the claims 1 to 10 for producing of pharmaceutical compositions as vector for the in vivo and ex vivo transfection of cells.

19. Use of liposomes of one of the claims 1 to 10 for producing vectors for the in vitro transfection of cells.

**Revendications**

1. Liposomes amphotères, **caractérisés en ce que** les liposomes comprennent au moins un porteur de charge positif et au moins un porteur de charge négatif différent, moyennant quoi les liposomes ont un point isoélectrique entre 4 et 8 et les liposomes comprennent un lipide neutre, sélectionné dans le groupe constitué de la phosphatidylcholine, le phosphatidyléthanolamine, le cholestérol, les tétraétherlipides, le céramide, les sphingolipides et/ou le diacylglycérol.

**2.** Liposomes amphotères selon la revendication 1, **caractérisés en ce que** les liposomes ont un point isoélectrique compris entre 5 et 7.

**3.** Liposomes amphotères, **caractérisés en ce que** les liposomes comprennent au moins un porteur de charge amphotère, moyennant quoi le porteur de charge amphotère a un point isoélectrique compris entre 4 et 8, et les liposomes comprennent un lipide neutre sélectionné dans le groupe constitué de la phosphatidylcholine, le phosphatidyléthanolamine, le cholestérol, les tétraétherlipides, le céramide, les sphingolipides et/ou le diacylglycérol.

**4.** Liposomes amphotères selon la revendication précédente, **caractérisés en ce que** le porteur de charge amphotère a un point isoélectrique compris entre 5 et 7.

**5.** Liposomes amphotères, **caractérisés en ce que** les liposomes comprennent au moins un porteur de charge amphotère ayant un point isoélectrique entre 4 et 8, et un porteur de charge anionique et/ou cationique, moyennant quoi les liposomes comprennent un lipide neutre sélectionné dans le groupe constitué de la phosphatidylcholine, le phosphatidyléthanolamine, le cholestérol, les tétraétherlipides, le céramide, les sphingolipides et/ou le diacylglycérol.

**6.** Liposomes amphotères selon la revendication 5, **caractérisés en ce que** les liposomes ont un point isoélectrique compris entre 5 et 7.

**7.** Liposomes amphotères selon l'une des revendications précédentes, **caractérisés en ce** les liposomes ont une grandeur moyenne comprise entre 50 et 1000 nm, de préférence entre 70 et 250 nm et de manière encore plus préférée entre 60 et 130 nm.

**8.** Liposomes amphotères selon l'une des revendications précédentes, **caractérisés en ce que** les liposomes comprennent un principe actif.

**9.** Liposomes amphotères selon la revendication précédente,
**caractérisés en ce que** le principe actif est une protéine, un peptide, un ADN, un ARN, un nucléotide antisens et/ou un nucléotide decoy.

**10.** Liposomes amphotères selon l'une des revendications précédentes, **caractérisés en ce qu'**au moins 80 % du principe actif se trouvent à l'intérieur du liposome.

**11.** Procédé de charge de principe actif dans des liposomes selon l'une des revendications 1 à 10, **caractérisé en ce qu'**on utilise un pH défini pour encapsuler et qu'on règle un second pH pour séparer le matériau non lié.

**12.** Procédé de charge de principe actif dans des liposomes selon l'une des revendications 1 à 10, **caractérisé en ce qu'**on rend perméable les liposomes à un pH défini et qu'on les obture.

**13.** Utilisation des liposomes selon l'une des revendications 1 à 10 pour préparer des nanocapsules.

**14.** Utilisation des liposomes selon l'une des revendications 1 à 10 pour préparer des systèmes de libération pour le diagnostic.

**15.** Utilisation des liposomes selon l'une des revendications 1 à 10 pour préparer des préparations pharmaceutiques destinées au transport et/ou à la libération des principes actifs.

**16.** Utilisation des liposomes selon l'une des revendications 1 à 10 pour préparer des préparations pharmaceutiques sous forme de formulation de retard ou de forme retard circulant.

**17.** Utilisation des liposomes selon l'une des revendications 1 à 10 pour préparer une préparation pharmaceutique destinée à une administration par voie intraveineuse et/ou péritonéale.

**18.** Utilisation des liposomes selon l'une des revendications 1 à 10 pour préparer des préparations pharmaceutiques servant de vecteur de transfection de cellules in vivo et ex vivo.

**19.** Utilisation des liposomes selon l'une des revendications 1 à 10 pour préparer des vecteurs de transfection de cellules

in vitro.

**Figur 1A**

Transfektion von HeLa-Zellen mit amphoteren Liposomen (POPC/DOTAP/CHEMS 60/30/10)

**Figur 1B**

Transfektion von CHO-Zellen mit amphoteren Liposomen (POPC/DOTAP/CHEMS 60/30/10)